(19) Europäisches Patentamt
European Patent Office
Office européen des brevets

(11) Veröffentlichungsnummer: **0 182 247**
**A1**

(12) # EUROPÄISCHE PATENTANMELDUNG

(21) Anmeldenummer: **85114340.4**

(22) Anmeldetag: **12.11.85**

(51) Int. Cl.⁴: **C 07 D 215/22**, A 61 K 31/495

(30) Priorität: **22.11.84 DE 3442570**

(43) Veröffentlichungstag der Anmeldung: **28.05.86**
**Patentblatt 86/22**

(84) Benannte Vertragsstaaten: **AT BE CH DE FR GB IT LI LU NL SE**

(71) Anmelder: **HOECHST AKTIENGESELLSCHAFT,**
**Postfach 80 03 20, D-6230 Frankfurt am Main 80 (DE)**

(72) Erfinder: **Lattrell, Rudolf, Dr., Heuhohlweg 6h,**
**D-6240 Königstein/Taunus (DE)**
Erfinder: **Klatt, Peter, Dr., Theresenstrasse 51,**
**D-6233 Kelkheim (Taunus) (DE)**
Erfinder: **Gerhards, Hermann, Dr., Wacholderweg 4,**
**D-6238 Hofheim am Taunus (DE)**
Erfinder: **Bauer, Fritz, Dr., Oranienstrasse 46,**
**D-6232 Bad Soden am Taunus (DE)**

(54) **Neue substituierte Phenylpiperazinderivate, Verfahren zu deren Herstellung und die Verwendung suubstituierter Phenylpiperazinderivate als Aggressionshemmer für Tiere.**

(57) Neue substituierte Phenylpiperazinderivate der Formel I

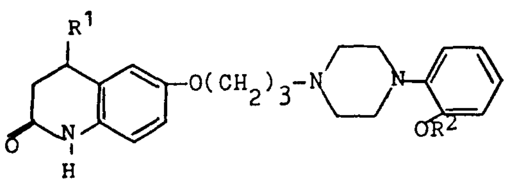

(I)

worin $R^1$ und $R^2$ die angegebene Bedeutung haben, sowie deren physiologisch verträgliche Salze und Verfahren zu ihrer Herstellung werden beschrieben. Die Verbindungen zeigen bei Tieren beruhigende und aggressionshemmende Wirkung. Ferner werden Verbindungen der Formel I'

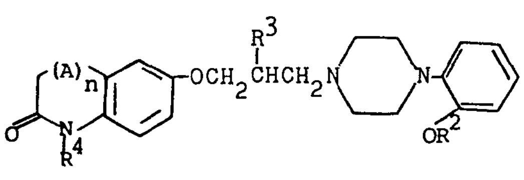

(I')

worin n, A, $R^2$, $R^3$ und $R^4$ die angegebenen Bedeutungen haben, sowie deren physiologisch verträgliche Salze zur Beruhigung und Aggressionshemmung bei Tieren beanspruch.

HOECHST AKTIENGESELLSCHAFT HOE 84/F 279 Dr.D/cr

Neue substituierte Phenylpiperazinderivate, Verfahren zu deren Herstellung und die Verwendung substituierter Phenylpiperazinderivate als Aggressionshemmer für Tiere

Die Erfindung betrifft neue substituierte Phenylpiperazinderivate der Formel I

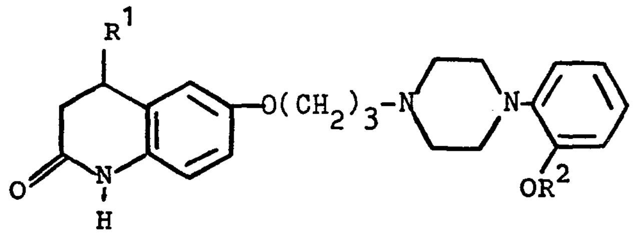

(I)

sowie deren physiologisch verträgliche Salze. Die Verbindungen bewirken bei Tieren eine Aggressionshemmung und finden daher als Aggressionshemmer bei Tieren Verwendung.

In der Formel bedeuten
$R^1$ Wasserstoff oder Phenyl und
$R^2$ $C_1$-$C_6$ Alkyl, wobei die gleichzeitige Bedeutung $R^1$ = Wasserstoff und $R^2$= Methyl ausgenommen ist.

$R^2$ bedeutet vorzugsweise Methyl, Äthyl, Propyl, Isopropyl oder n-Butyl. Besonders bevorzugt ist die Verbindung worin $R^1$ Wasserstoff und $R^2$ Äthyl bedeuten.

Die Erfindung betrifft weiterhin ein Verfahren zur Herstellung von Verbindungen der Formel I, das dadurch gekennzeichnet ist, daß man

a) eine Verbindung der Formel II,

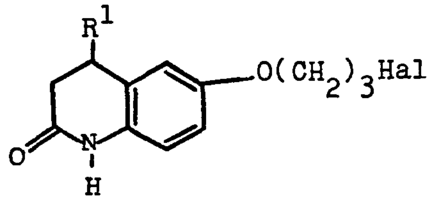

(II)

worin $R^1$ die obengenannte Bedeutung hat und Hal ein Halogenatom bedeutet, mit einem Phenylpiperazin der Formel III,

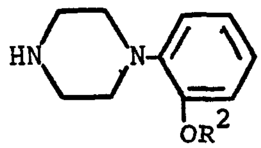

(III)

worin $R^2$ die obengenannte Bedeutung hat, umsetzt, oder

b) eine Verbindung der Formel IV,

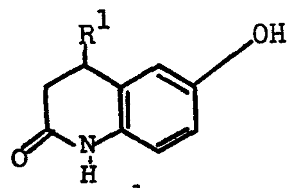

(IV)

worin $R^1$ die obengenannte Bedeutung hat, mit einer Verbindung der Formel V,

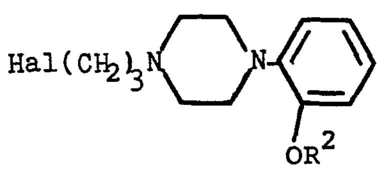

(V)

worin Hal und $R^2$ die obengenannte Bedeutung haben, umsetzt, oder

c) eine Verbindung der Formel VI

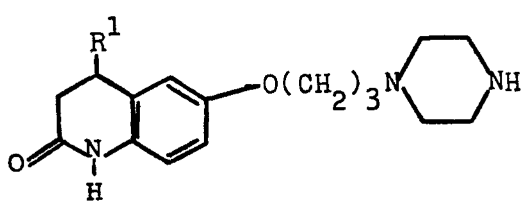

(VI)

worin $R^1$ die obengenannte Bedeutung hat, mit einer Verbindung der Formel VII,

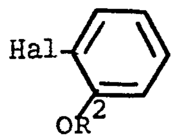

(VII)

worin Hal und $R^2$ die obengenannte Bedeutung haben, umsetzt oder

d) eine Verbindung der Formel VIII

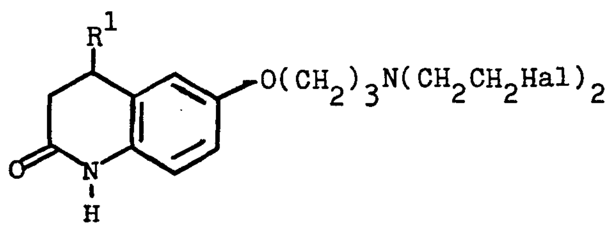

(VIII)

worin Hal und $R^1$ die obengenannte Bedeutung haben, mit einer Verbindung der Formel IX,

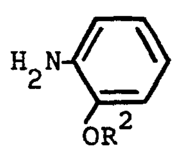

(IX)

worin $R^2$ die obengenannte Bedeutung hat, umsetzt oder

e) eine Verbindung der Formel X,

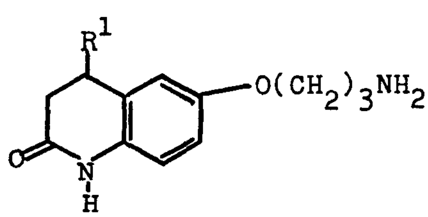

(X)

worin $R^1$ die obengenannte Bedeutung hat, mit einer Verbindung der Formel XI,

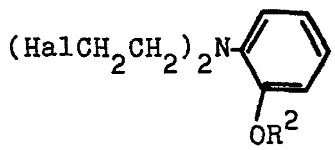
(XI)

worin Hal und $R^2$ die obengenannte Bedeutung haben, umsetzt und die Reaktionsprodukte gegebenenfalls in die physiologisch verträgichen Salze überführt.

Nach dem Verfahren a wird die Umsetzung der 3-Halopropoxyverbindung der Formel II, die in an sich bekannter Weise aus der Verbindung der Formel IV und 1,3-Dihalopropanen erhalten wird, mit einem Phenylpiperazin der Formel III vorzugsweise in Gegenwart einer Base wie Natrium- der Kaliumhydroxid, Natrium- oder Kaliumcarbonat, Natriumhydrid,tertiären Aminen, wie Triäthylamin, oder Pyridin durchgeführt, jedoch kann auch in Abwsenheit einer Base gearbeitet werden. Die Reaktion wird im allgemeinen bei Temperaturen zwischen 50° und 200°C, vorzugsweise zwischen 60° und 160°C ausgeführt unter Verwendung von äquimolaren Mengen bis zu einem fünffach molaren Überschuß, vorzugsweise von äquimolaren Mengen, des Phenylpiperazins der Formel III. Werden Lösungsmittel verwendet, so kommen beispielsweise Äther wie Tetrahydrofuran oder Dioxan, Glykoläther wie Diglym, Ketone wie Aceton oder Methyläthylketon, aromatische Kohlenwasserstoffe wie Toluol, Chlorbenzol, Alkohole wie Äthanol, Isoamylalkohol, aprotische Lösungsmittel, wie Dimethylformamid, Dimethylacetamid oder dergleichen in Frage.

Nach dem Verfahren b wird die Umsetzung der phenolischen Verbindung der Formel IV mit Phenylpiperazinderivaten der

Formel V in an sich bekannter Weise ausgeführt, wobei vorzugsweise die vorstehend beschriebenen Reaktionsbedingungen angewendet werden. Eine bevorzugte Verfahrensvariante besteht darin, daß man zunächst die phenolische Verbindung der Formel IV mittels eines Alkalialkoholates oder eines Alkalihydrids in das entsprechende Alkalisalz überführt.

Nach dem <u>Verfahren c</u> werden die monosubstituierten Piperazinderivate der Formel VI, die ihrerseits nach der Verfahrensvariante a) aus Verbindungen der allgemeinen Formel II und Piperazin erhalten werden, mit Verbindungen der Formel VII kondensiert. Die Umsetzung wird vorzugsweise in einem polaren Lösungsmittel, z.B. Alkoholen wie Isoamylalkohol, Äthern wie Diglym oder aprotischen Lösungsmittel wie Dimethylformamid, bei Temperaturen zwischen 60° und 200°C in Gegenwart eines Akzeptors für die im Verlauf der Reaktion gebildete Halogenwasserstoffsäure, beispielsweise Kaliumcarbonat, ausgeführt.

Nach dem <u>Verfahren d</u> wird die Kondensation der Verbindungen VIII mit Anilinderivaten der Formel IX in einem Lösungsmittel, wie sie vorstehend genannt wurden, bei einer Temperatur zwischen 60 und 160°C, vorzugsweise in Gegenwart eines Halogenwasserstoffakzeptors, wie z.B. Kaliumcarbonat oder Pyridin, ausgeführt.

Nach dem <u>Verfahren e</u> werden Aminopropylverbindungen der Formel X, die ihrerseits in an sich bekannter Weise aus Verbindungen der Formel II mit alkoholischer Ammoniaklösung erhalten werden, mit N-(Bis-Halogenäthyl)anilinen der Formel XI kondensiert, wobei zweckmäßigerweise die für die Verfahren c und d beschriebenen Reaktionsbedingungen angewendet werden.

Die erfindungsgemäßen Verbindungen der Formel I werden in freier Form oder als Salze isoliert, je nach den angewandten Reaktionsbedingungen. Die freie Base kann nach Reaktion mit anorganischen oder organischen Säuren in ihre pharmakologisch verträglichen Salze übergeführt werden. Solche Säuren sind z.B. Salzsäure, Schwefelsäure, Phosphorsäure, aliphatische, alicyclische, araliphatische, aromatische oder heterocyclische Carbonsäuren oder Sulfonsäuren wie Essigsäure, Weinsäure, Milchsäure, Maleinsäure, Fumarsäure, Zitronensäure, Oxalsäure, Methansulfonsäure, Hydroxyäthansulfonsäure oder synthetische Harze, die saure Gruppen enthalten.

Phenylpiperazinderivate, welche in ortho-Stellung des Phenylrestes substituiert sind, wurden bereits beschrieben (vergl. Europäische Patentschrift 0 005 828 bzw. US-PS 4 234 584). Die in diesen Schriften beschriebenen Verbindungen zeigen neuroleptische Wirkung.

Es wurde nun überraschend gefunden, daß die Phenylpiperazinopropyloxychinolinone der Formel I eine beruhigende und insbesondere aggressionshemmende Wirkung bei Tieren besitzen. Ferner wurde gefunden, daß auch in der US-PS 42 34 584 beschriebene Phenylpiperazinderivate eine solche Wirkung zeigen.

Die Erfindung betrifft daher auch Verbindungen der Formel I'

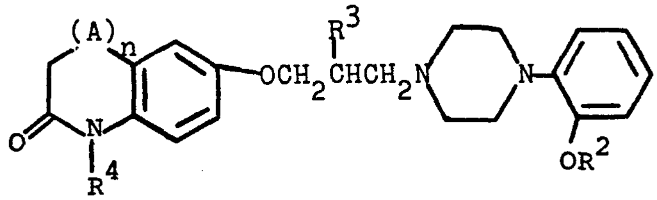

(I')

worin

n = 0 oder 1

A = $CH-R^1$, wobei $R^1$ Wasserstoff oder Phenyl bedeutet

$R^2$= $C_1$-$C_6$-Alkyl

$R^3$= Wasserstoff oder, falls n = 0 ist, auch Hydroxy

$R^4$= Wasserstoff oder Methyl

bedeuten und deren physiologisch verträgliche Salze zur Beruhigung und Aggressionshemmung bei Tieren.

Die Erfindung betrifft ferner pharmazeutische Präparate enthaltend eine Verbindung der Formel I oder deren Salze, insbesondere zur Beruhigung und Aggressionshemmung bei Tieren, vorzugsweise bei Schweinen.

Insbesondere betrifft die Erfindung Präparate, die als Wirkstoff eine Verbindung der Formel I enthalten, worin $R^1$ Wasserstoff und $R^2$ Äthyl bedeuten.

Die Wirkung der in der Tabelle 1 aufgeführten Verbindungen an Haustieren wird im folgenden beschrieben:

<u>Tabelle 1</u>

Orthosubstituierte Phenylpiperazinderivate 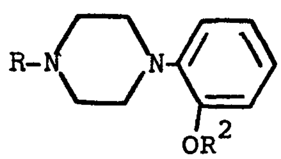

| Verbindung | R | $R^2$ |
|---|---|---|
| 1 | 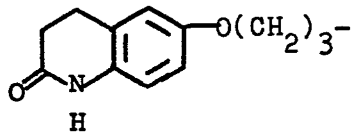 | $C_2H_5$ |
| 2 | " | $CH(CH_3)_2$ |
| 3 | " | $C_4H_9$ |
| 4 | 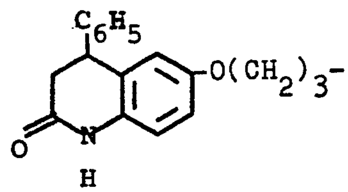 | $CH_3$ |
| 5 | 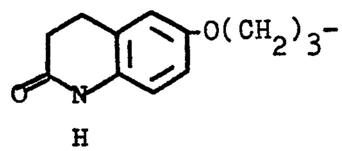 | $CH_3$ |
| 6 | 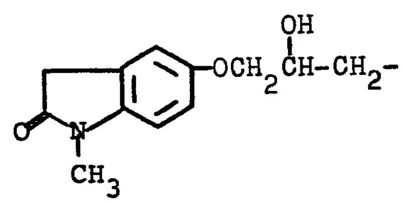 | $CH_3$ |
| 7 | " | $C_2H_5$ |
| 8 | 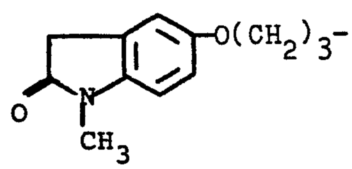 | $CH_3$ |

In Untersuchungen an verschiedenen Tierarten zeigte sich, daß die genannten Verbindungen die Affektivität und Dynamik psychischer Prozesse dämpfen ohne gleichzeitig hypnotische oder narkotische Wirkungen zu entfalen. Aggressives Verhalten von Tieren gegenüber Artgenossen oder dem Menschen wird nach Verabreichung der Wirkstoffe der Formel I' unter allgemeiner Beruhigung gedämpft. Die Bewegungsaktivität sowie Ansprechbarkeit der Tiere bleibt dabei weitgehend erhalten.

Prüfung am Schwein

Schweine, die in Gruppen neu auf- oder umgestallt werden, tragen nach dem Zusammensetzen untereinander Rangordnungskämpfe aus. Es kommt dabei zu Verletzungen, Gewichtsverlusten und einer erheblichen Stress-Belastung, als deren Folge nicht selten Todesfälle auftreten. Werden solche Schweine vor dem Zusammensetzen mit einer der beschriebenen Verbindungen oral oder intramuskulär behandelt, so treten die geschilderten Rangordnungskämpfe nicht oder nur vereinzelt in leichter Form auf. Die nachfoglende Tabelle 2 gibt die Prüfungsergebnisse wieder, wobei sich der Beobachtungszeitraum auf 5 - 7 Stunden nach der Behandlung erstreckt.

Tabelle 2

| Verbindung Nr. der Tabelle 1 | Dosis mg/kg | Appl.-Art | Zahl der behandelten Schweine | Kämpfe pro Tier | Zahl der unbehandelten Schweine | Kämpfe pro Tier |
|---|---|---|---|---|---|---|
| 1 | 2,5 | per os | 12 | 0,5 | 12 | 1,3 |
| | 5,0 | per os | 28 | 0,5 | 28 | 4,2 |
| | 0,25 | i.m. | 14 | 0,6 | 12 | 2,7 |
| | 0,5 | i.m. | 9 | 1,8 | 8 | 5,6 |
| | 1,0 | i.m. | 18 | 0,8 | 18 | 2,5 |
| | 1,0 | i.m. | 3 | 0 | 3 | 2,3 |
| 2 | 1,0 | i.m. | 3 | 1,7 | 3 | 3,4 |
| 3 | 1,0 | i.m. | 3 | 0 | 3 | 2,8 |
| 4 | 1,0 | i.m. | 8 | 0,6 | 9 | 2,8 |
| 5 | 1,0 | i.m. | 8 | 0,6 | 9 | 2,8 |
| 6 | 1,0 | i.m. | 5 | 0 | 5 | 2,4 |
| 7 | 1,0 | i.m. | 3 | 0,7 | 3 | 2,8 |
| 8 | 1,0 | i.m. | 7 | 0 | 3 | 2,4 |

Prüfung am Rind

Zwei widersetzliche und nur schwer zu führende dreijährige Bullen wurden nacheinander vom Anbindestand mit Sichtblenden von drei erfahrenen Pflegern zu einer ca. 50 m entfernten Großtierwaage geführt. Dort wurde den Tieren einmal 0,5 mg/kg KGW und im anderen Fall 1,0 mg/kg KGW der Verbindung nach Beispiel 1 i.m. verabreicht. Nach 15 Minuten konnten beide Bullen ohne Sichtblende von jeweils einem Pfleger mühelos zum Anbindestand zurückgeführt werden. Die beruhigende und antiaggressive Wirkung war nach ca. 5 Stunden abgeklungen.

Prüfung am Hund

Die Verbindung nach Beispiel 1 wurde jeweils drei ♀ Beagle-Hunden (1 Jahr alt) in Dosierungen von 0,25; 0,5 und 1,0 mg/kg KGW i.m. verabreicht. Anschließend wurden die Tiere in einen ihnen unbekannten Raum verbracht und das Verhalten bis 5 Stunden post appl. ständig beobachtet.

Dosis 0,25 mg/kg: Bis 10 Minuten nach der Behandlung entsprach das Verhalten der Norm (Erkundung der neuen Umgebung). Anschließend zeigten die Tiere für ca. 30 Minuten geringe Unsicherheiten in der Bewegung, erschienen sediert und legten sich zusammen nieder. Die Reaktion auf Fremdgeräusche war erhalten. Nach 1 Stunde wechselten Ruhe- und Aktivitätsphasen ab, wobei die beruhigende Wirkung bis 3 Stunden post appl. abgeklungen war.

Dosis 0,5 mg/kg: 5 Minuten nach der Behandlung traten deutliche Unsicherheiten in der Bewegung auf, die Tiere wirkten stark sediert und legten sich nieder. Die Ansprechbarkeit blieb erhalten. Nach 45 Minuten war der Bewegungsablauf wieder koordiniert und Ruhe- sowie

Aktivitätsphasen wechselten sich ab. Die beruhigende Wirkung hielt bis 3 Stunden an.

Dosis 1,0 mg/kg: Innerhalb von 3-5 Minuten nach der Behandlung war eine stark ausgeprägte Beruhigung eingetreten. Die Tiere konnten sich nur mit Mühe auf den Beinen halten. Teilweise standen sie mit gesenktem Kopf und ließen ansatzweise Stereotypien erkennen. Abgelegte Hunde, (Hunde, die sich ohne Fremdhilfe hingelegt haben) die in Rückenposition gebracht wurden, verharrten in dieser Stellung für längere Zeit. Nach 30 Minuten war die Ansprechbarkeit wieder hergestellt. Es schlossen sich lange Ruhephasen an, die nach 3 Stunden mit Aktivitätsphasen wechselten, wobei der Beweungsablauf der Norm entsprach. Nach 4 Stunden begann die beruhigende Wirkung abzuklingen und war nach 5 Stunden fast vollständig aufgehoben.

Bei allen drei Dosierungen wurde während der Beobachtungszeit keine Aggression gegenüber Artgenossen festgestellt.

Prüfung an der Katze

Drei zusammengehaltene ♂ kastrierte Katzen (Alter 6 Jahre) wurden mit der Verbindung nach Beispel 1 in einer Dosis von 1,0 mg/kg KGWQ i.m. behandelt und in ihrer Box belassen. Die Beobachtungszeit betrug 5 Stunden. 10 Minuten post appl. trat eine deutliche Beruhigung ein. Die Tiere zeigten Unsicherheiten im Bewegungsablauf und legten sich nieder. Die Ansprechbarkeit war für 1 Stunde herabgesetzt. Die vor Behandlung nicht handzahmen Katzen ließen sich mühelos auf den Tisch setzen und verschiedene Untersuchungen ohne Abwehrbewegungen durchführen. Ein geringes Fluchtverhalten war erkennbar. Nach 4 Stunden flachte die beruhigende Wirkung ab und war nach 5 Stunden fast vollständig aufgehoben.

Aus den Prüfungsergebnissen geht hervor, daß die Verbindungen bei Tieren dosisabhängig nach oraler und parenteraler Applikation eine gute beruhigende und darüberhinaus auch antiaggresive Wirkung entfalten.

Die folgenden Ausführungsbeispiele für erfindungsgemäß herstellbare Verbindungen der Formel I dienen zur weiteren Erläuterung der Erfindung, schränken sie jedoch nicht darauf ein.

Beispiel 1

6-[3-(4-Ethoxyphenyl)-1-piperazinyl)-propyloxy]-1,2,3,4-tetrahydrochinolinon-2

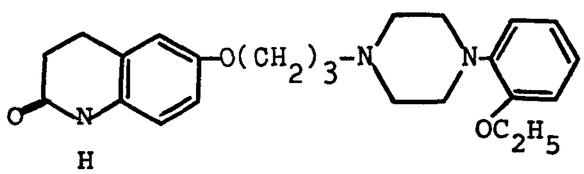

Verfahren a:

Stufe 1: Darstellung von 6-(3-Brompropyloxy)-1,2,3,4-tetrahydrochinolinon-2:

Ein Gemisch aus 12,6 g (0,076 mol) 6-Hydroxy-1,2,3,4-tetrahydrochinolinon-2, 15.8 (0.115 mol) Kaliumcarbonat (wasserfrei, gepulvert), 46.5 g (0.23 mol) 1.3-Dibrompropan und 40 ml N,N-Dimethylformamid wird 20 Stunden bei Raumtemperatur gerührt. Es werden 200 ml Wasser und 100 ml Petroläther zugegeben, nach 1 Stunde Rühren wird der gebildete Niederschlag abgesaugt, mit Petroläther gewaschen und getrocknet.
Ausbeute 18.5 g, Schmp. 100-106°C. Nach Umkristallisieren aus Ethanol werden 12.6 g vom Schmp. 116-117 erhalten.

Stufe 2:

Ein Gemisch aus 21.2 g (0.074 mol) 6-(3-Brompropyloxy)-1,2,3,4-tetrahydrochinolinon-2, 15.3 g (0.074 mol) 1-(2-Ethoxyphenyl)-piperazin, 31.2 g (0,223 mol) wasserfreiem, gepulvertem Kaliumcarbonat, 1.9 g (0.011 mol) Kaliumjodid und 180 ml Toluol wird 23 Stunden unter Rückfluß erhitzt. Nach dem Erkalten werden 200 ml Wasser zugegeben, die Phasen getrennt und die organische Phase zweimal mit Wasser gewaschen. Das Lösungsmittel wird im Vakuum entfernt und der Rückstand mit Äther verrieben. Die kristalline freie Base schmilzt bei 153-154°C. Es wird in Aceton gelöst und ein Überschuß äthanolischer Salzsäure zugegeben. Der gebildete Niederschlag wird abgeaugt, mit Aceton gewaschen und getrocknet.
Ausbeute: 25.1 g (83 % der Theorie), Dihydrochlorid vom Schmp. 216°C.

Verfahren b

Zu 1,63 g (0.01 mol) 6-Hydroxy-1,2,3,4-tetrahydrochinolinon-2 in 20 ml Dioxan werden 0.01 mol Natriumhydrid und nach Ende der Gasentwicklung 2.8 g (0.01 mol) 1-Chloro-3-[4-(2-ethoxyphenyl)-1-piperazinyl]-propan gegeben. Die Mischung wird 10 Stunden unter Rückfluß erhitzt, nach dem Erkalten mit Wasser verdünnt und mit Methylendichlorid extrahiert. Nach dem Einengen wird mit Äther versetzt, der Niederschlag abgesaugt, mit Äther gewaschen und getrocknet. Schmp. 153-154°C, identisch mit der nach Verfahren a) erhaltenen Verbindung.

Verfahren c

Ein Gemisch aus 2.9 g (0.01 mol) 6-[3-(1-Piperazinyl)-propyloxy]-1,2,3,4-tetrahydrochinolinon-2, 1.4 g (0.01

mol) 2-Ethoxyfluorbenzol, 4 g Kaliumcarbonat und 50 ml N,N-Dimethylformamid wird 24 Stunden unter Rückfluß gehalten. Nach dem Erkalten wird filtriert, das Lösungsmittel im Vakuum entfernt, der Rückstand über 200 g Kieselgel mit Methylenchlorid / Methanol (4:1) chromatographiert. Der Rückstand aus den Produktfraktionen wird mit Äther verrieben. Die Verbindung vom Schmp. 152-154°C ist identisch mit der nach Verfahren a) erhaltenen Verbindung.

Verfahren d:

3.5 g (0.01 mol) 6-[3-(Bis-(2-chloroäthyl)-amino)-propyloxy]-1,2,3,4-tetrahydrochinolinon-2 und 4.1 g (0.03 mol) o-Ethoxyanilin werden in 30 ml Diglym 10 Stunden auf 150°C erhitzt. Die Mischung wird mit Wasser verdünnt, mit Methylenchlorid extrahiert und das Lösungsmittel im Vakuum entfernt. Aus dem Rückstand wird wie vorstehend beschrieben durch Chromatographie eine kristalline Verbindung isoliert, die in allen Eigenschaften mit der nach Verfahren a), b) bzw. c) erhaltenen identisch ist.

Verfahren e:

Eine Mischung aus 2.3 g (0.01 mol) 6-(3-Amino-propyloxy)-1,2,3,4-tetrahydrochinolinon-2, 2,6 g (0.01 mol) Bis-N,N-(2-chloroäthyl)-2-ethoxy-anilin, 5 g wasserfreiem Kaliumcarbonat und 30 ml N,N-Dimethylformamid wird 10 Stunden auf 130°C erhitzt. Nach dem Erkalten wird mit Wasser verdünnt und wie vorstehend beschrieben aufgearbeitet. Die Verbindung ist mit der nach Verfahren a erhaltenen identisch.

Die Verbindungen der Tabelle 3 werden in Analogie zu Beispiel 1, Verfahren a, aus 6-(3-Brompropyloxy)-1,2,3,4-tetrahydrochinolinon-2 bzw. 6-(3-Brompropyloxy)-4-phenyl-

tetrahydrochinolinon-2 (Beispiel 4) und den entsprechenden Phenylpiperazinderivaten erhalten.

Tabelle 3

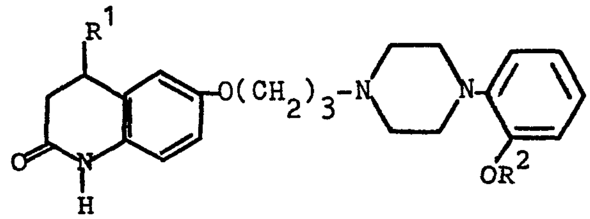

| Beisp. Nr. | $R^1$ | $R^2$ | Base (Schmp.°C) | Dihydrochlorid (Schmp.°C) | Ausbeute % d.Th. |
|---|---|---|---|---|---|
| 2 | H | $CH(CH_3)_2$ | 136-138 | 187-189 | 89 |
| 3 | H | $C_4H_9$-n | 126-127 | 216-217 | 90 |
| 4 | $C_6H_5$ | $CH_3$ | 160-161 | 220-222 | 81 |

Die Verbindungen der Tabelle 4 sind in der US-PS 4 234 584 beschrieben und wurden entsprechend den Angaben in den Beispielen 35, 1, 12 und 25 erhalten.

Tabelle 4

Beispiel

5. 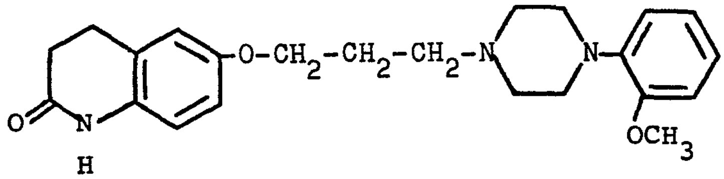

6. 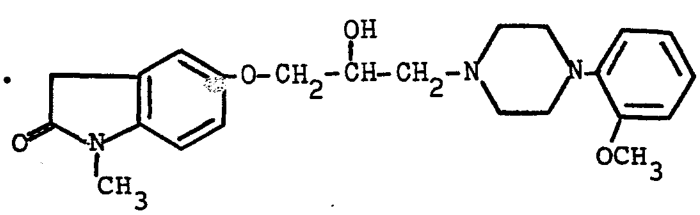

7. 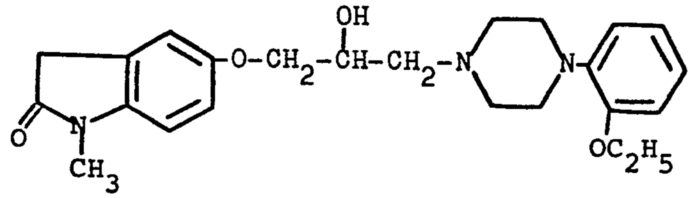

8. 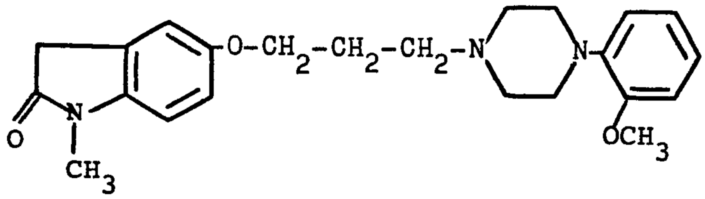

PATENTANSPRÜCHE

1. Phenylpiperazinderivat der Formel I

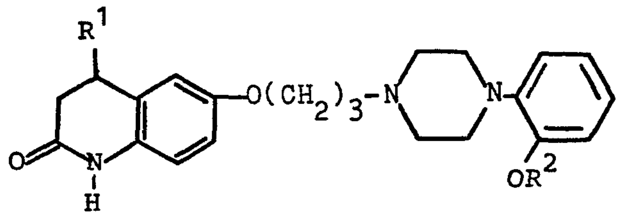

(I)

worin bedeuten

$R^1$= Wasserstoff oder Phenyl

$R^2$= $C_1$-$C_6$ Alkyl, wobei die gleichzeitige Bedeutung $R^1$ = Wasserstoff und $R^2$= Methyl ausgenommen ist, und deren physiologisch verträgliche Salze.

2. Phenylpiperazinderivat gemäß Anspruch 1, dadurch gekennzeichnet, daß in Formel I $R^1$ Wasserstoff und $R^2$ Äthyl bedeuten.

3. Verwendung von Verbindungen der Formel I'

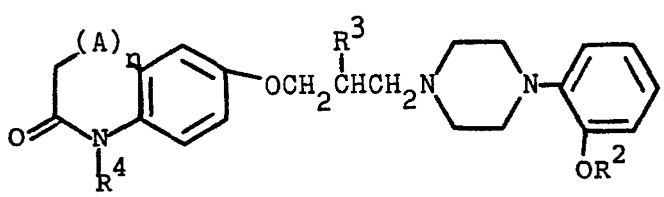

(I')

worin

n = 0 oder 1

A = CH-$R^1$, wobei $R^1$ Wasserstoff oder Phenyl bedeutet

$R^2$= $C_1$-$C_6$-Alkyl

$R^3$= Wasserstoff oder, falls n = 0 ist, auch Hydroxy

$R^4$= Wasserstoff oder Methyl

bedeuten oder deren physiologisch verträglichen Salzen zur Herstellung eines pharmazeutischen Präparates zur Beruhigung und Aggressionshemmung bei Tieren.

4. Verwendung von Verbindungen der Formel I gemäß Anspruch 1, sowie von deren physiologisch verträglichen Salzen zur Beruhigung und Aggressionshemmung bei Tieren.

5. Pharmazeutisches Präparat enthaltend eine Verbindung gemäß Anspruch 1.

6. Pharmazeutisches Präparat enthaltend eine Verbindung gemäß Anspruch 1 zur Beruhigung und Aggressionshemmung bei Tieren.

7. Pharmazeutisches Präparat zur Beruhigung und Aggressionshemmung bei Tieren enthaltend eine Verbindung der Formel I gemäß Anspruch 1, worin $R^1$ Wasserstoff und $R^2$ Äthyl bedeuten, oder ein physiologisch verträgliches Salz dieser Verbindung.

8. Verfahren zur Herstellung von Verbindungen gemäß Anspruch 1, dadurch gekennzeichnet, daß man

a) eine Verbindung der Formel II,

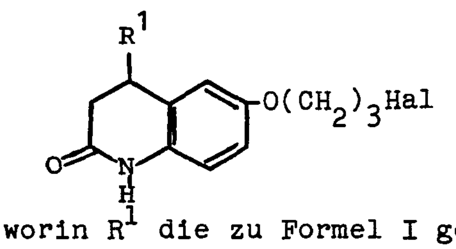

(II)

worin $R^1$ die zu Formel I genannte Bedeutung hat und Hal ein Halogenatom bedeutet, mit einem Phenylpiperazin der Formel III,

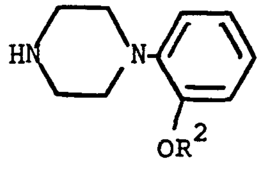

(III)

worin $R^2$ die obengenannte Bedeutung hat, umsetzt, oder

b) eine Verbindung der Formel IV,

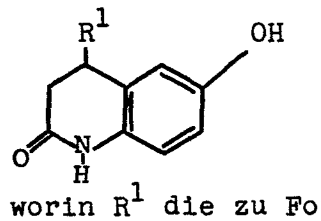

(IV)

worin $R^1$ die zu Formel I genannte Bedeutung hat, mit einer Verbindung der Formel V,

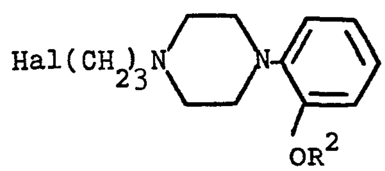

(V)

worin Hal und $R^2$ die obengenannte Bedeutung haben, umsetzt, oder

c) eine Verbindung der Formel VI

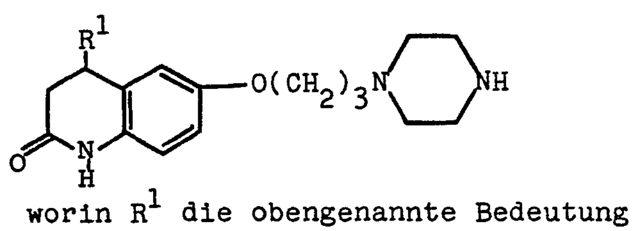

(VI)

worin $R^1$ die obengenannte Bedeutung hat, mit einer Verbindung der Formel VII,

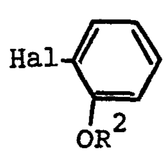

(VII)

worin Hal und $R^2$ die obengenannte Bedeutung haben, umsetzt, oder

d) eine Verbindung der Formel VIII

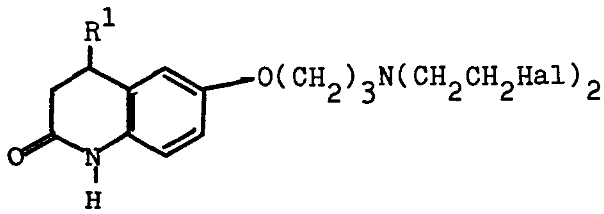

(VIII)

worin Hal und $R^1$ die obengenannte Bedeutung haben, mit einer Verbindung der Formel IX,

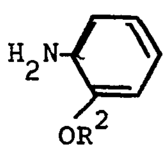

(IX)

worin $R^2$ die obengenannte Bedeutung hat, umsetzt, oder

e) eine Verbindung der Formel X,

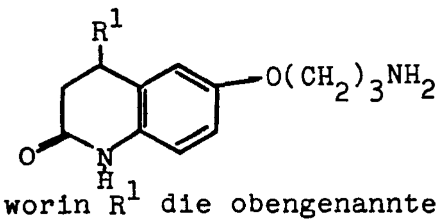

(X)

worin $R^1$ die obengenannte Bedeutung hat, mit einer Verbindung der Formel XI,

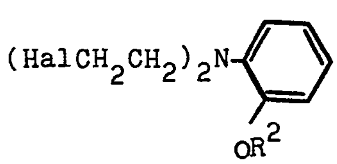

(XI)

worin Hal und $R^2$ die obengenannte Bedeutung haben, umsetzt und die Reaktionsprodukte gegebenenfalls in die physiologisch verträgichen Salze überführt.

PATENTANSPRÜCHE für Österreich

1. Verfahren zur Herstellung von Phenylpiperazinderivaten der Formel I

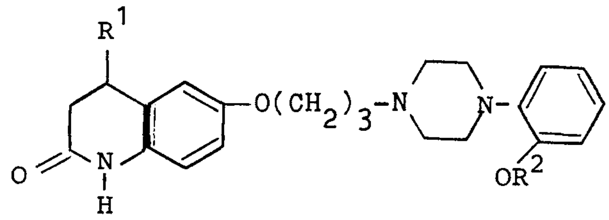

I

worin bedeuten
$R^1$= Wasserstoff oder Phenyl
$R^2$= $C_1$-$C_6$ Alkyl, wobei die gleichzeitige Bedeutung
$R^1$= Wasserstoff und $R^2$= Methyl ausgenommen ist, und von deren physiologisch verträglichen Salzen, dadurch gekennzeichnet, daß man

a) eine Verbindung der Formel II,

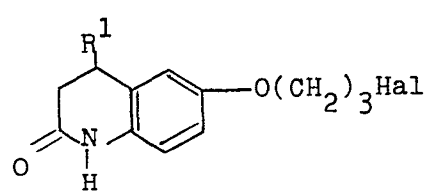

(II)

worin $R^1$ die zu Formel I genannte Bedeutung hat und Hal ein Halogenatom bedeutet, mit einem Phenyliperazin der Formel III,

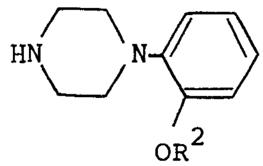

(III)

worin $R^2$ die obengenannte Bedeutung hat, umsetzt, oder

b) eine Verbindung der Formel IV,

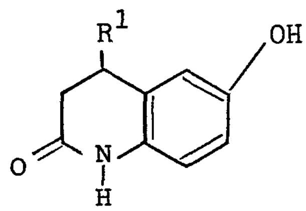

(IV)

worin $R^1$ die zu Formel I genannte Bedeutung hat, mit einer Verbindung der Formel V,

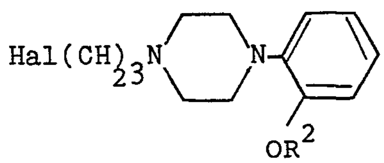

(V)

worin Hal und $R^2$ die obengenannte Bedeutung haben, umsetzt, oder

c) eine Verbindung der Formel VI

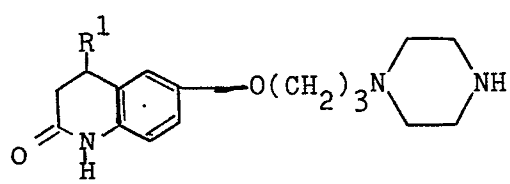

(VI)

worin $R^1$ die obengenannte Bedeutung hat, mit einer Verbindung der Formel VII,

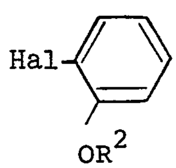

(VII)

worin Hal und $R^2$ die obengenannte Bedeutung haben, umsetzt, oder

d) eine Verbindung der Formel VIII

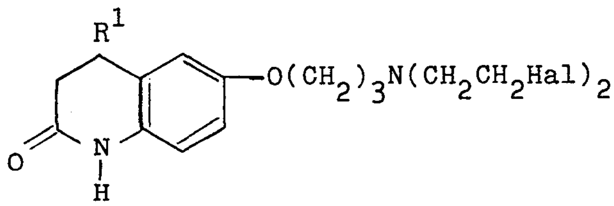

(VIII)

worin Hal und $R^1$ die obengenannte Bedeutung haben, mit einer Verbindung der Formel IX,

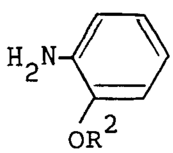

(IX)

worin $R^2$ die obengenannte Bedeutung hat, umsetzt, oder

e) eine Verbindung der Formel X,

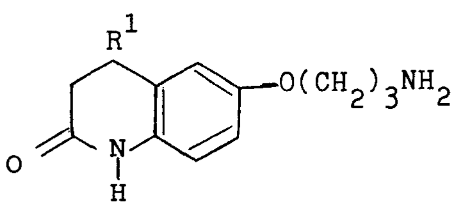

(X)

worin $R^1$ die obengenannte Bedeutung hat, mit einer Verbindung der Formel XI,

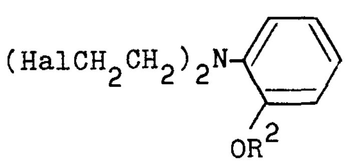

(XI)

worin Hal und $R^2$ die obengenannte Bedeutung haben, umsetzt und die Reaktionsprodukte gegebenenfalls in die physiologisch verträgichen Salze überführt.

2. Verfahren gemäß Anspruch 1, dadurch gekennzeichnet, daß man ein Phenylpiperazinderivat der Formel I worin $R^1$ Wasserstoff und $R^2$ Äthyl bedeuten, herstellt.

3. Verwendung von Verbindungen der Formel I'

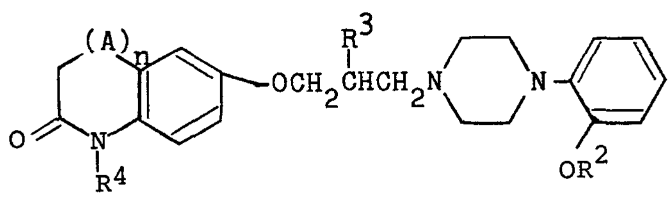

(I')

worin

n = 0 oder 1

A = $CH-R^1$, wobei $R^1$ Wasserstoff oder Phenyl bedeutet

$R^2$= $C_1-C_6$-Alkyl

$R^3$= Wasserstoff oder, falls n = 0 ist, auch Hydroxy

$R^4$= Wasserstoff oder Methyl

bedeuten oder deren physiologisch verträglichen Salzen zur Herstellung eines pharmazeutischen Präparates zur Beruhigung und Aggressionshemmung bei Tieren.

4. Verfahren zur Herstellung eines pharmazeutischen Präparates, dadurch gekennzeichnet, daß man eine Verbindung der im Anspruch 1 angegebenen Formel I oder ein physiologisch verträgliches Salz in eine geeignete Applikationsform bringt.

5. Verfahren gemäß Anspruch 4, dadurch gekennzeichnet, daß man eine Verbindung der Formel I gemäß Anspruch 1, worin $R^1$ Wasserstoff und $R^2$ Äthyl bedeuten, oder ein physiologisch verträgliches Salz dieser Verbindung in eine geeignete Applikationsform bringt.

Europäisches Patentamt

**EUROPÄISCHER TEILRECHERCHENBERICHT,**
der nach Regel 45 des Europäischen Patent-übereinkommens für das weitere Verfahren als europäischer Recherchenbericht gilt

Nummer der Anmeldung
EP 85114340.4

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | GB - A - 2 017 701 (OTSUKA)<br>* Ansprüche 1,32,35,36,41,43 *<br>-- | 1,2,5,8 | C 07 D 215/22<br>A 61 K 31/495 |
| D,Y | US - A - 4 234 584 (LATTRELL)<br>* Ansprüche 1,2; Beispiel 35; Spalte 1, Zeile 68 - Spalte 3, Zeile 28 *<br>-- | 1,2,5,8 | |
| X | DE - A1 - 2 853 996 (HOECHST)<br>* Ansprüche 1,4,5 *<br>---- | 3 | |

RECHERCHIERTE SACHGEBIETE (Int. Cl.4)
C 07 D 215/00

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-3,5-8
Unvollständig recherchierte Patentansprüche: -
Nicht recherchierte Patentansprüche: 4
Grund für die Beschränkung der Recherche:

Verfahren zur therapeutischen Behandlung des menschlichen oder tierischen Körpers. Art. 52(4), EPÜ

| Recherchenort | Abschlußdatum der Recherche | Prüfer |
|---|---|---|
| WIEN | 19-02-1986 | HOCHHAUSER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument